# EUROPEAN PATENT APPLICATION

(11) **EP 4 772 177 A1**
(43) Date of publication of application: **08.07.2026**
(21) Application number: 24859885.6
(22) Date of filing: 29.08.2024
(51) Int. Cl.: A61K 31/201, A23K 20/158, A23L 33/12, A61P 21/00

(54) **MUSCLE ATROPHY INHIBITOR**

(30) Priority: 30.08.2023 JP 2023140522
(71) Applicant: National University Corporation Kobe University, Kobe-shi, Hyogo 657-8501 (JP); Noster Inc., Muko-shi, Kyoto 617-0006 (JP)
(72) Inventor: OGAWA, Wataru, Kobe-shi, Hyogo 657-8501 (JP); HIRATA, Yu, Kobe-shi, Hyogo 657-8501 (JP); NOMURA, Kazuhiro, Kobe-shi, Hyogo 657-8501 (JP); KITAO, Kohey, Muko-shi, Kyoto 617-0006 (JP)
(74) Representative: Weickmann & Weickmann PartmbB
(86) International application number: PCT/JP2024/030918
(87) International publication number: WO 2025/047856

(57) **Abstract**

The present invention provides, as an embodiment thereof, a muscle atrophy inhibitor containing a long-chain hydroxylated fatty acid as an active ingredient.

## Description

### [Technical Field]

The present invention relates to long-chain hydroxylated fatty acid that inhibits muscle atrophy and is useful in the fields of medicament, food, and the like.

### [Background Art]

In humans and animals, muscle mass decreases from middle age onward due to aging and lifestyle habits. As people age, sarcopenia, which is muscle atrophy due to aging, becomes a problem. Besides sarcopenia, muscle atrophy includes disuse muscle atrophy in which muscles atrophy due to immobility caused by cast immobilization or bed rest, myopathy such as neuromuscular diseases, and the like, where independent activity is inhibited. When independent activity is inhibited by muscle atrophy, daily life is also affected, posing a significant social problem.

3-Hydroxyisovaleric acid is known to help maintain muscle mass, and as a substance that improves a unique flavor thereof and enhances its ability to suppress muscle atrophy, a composition containing 3-hydroxyisovaleric acid and an amino sugar has been reported (Patent Literature 1). In addition, there are a report that kynurenic acid, which is a tryptophan metabolite, can treat and prevent muscle atrophy-related diseases (Patent Literature 2), and a report on a muscle atrophy-inhibiting composition containing tripeptide as an active ingredient (Patent Literature 3). There is also a report that a gene expression regulator containing an odd-numbered fatty acid as an active ingredient inhibits muscle atrophy by suppressing the expression of the metallothionein-2 gene and enhancing the expression of the nucleoporin-210 gene (Patent Literature 4). Muscle atrophy has also been reported to be inhibited by suppressing the expression of chemokine CXCL10 by using a neutralizing antibody (Patent Literature 5).

Non Patent Literature 1 discusses nutrients involved in muscle synthesis, and describes that omega-3 fatty acids contribute to promoting muscle synthesis, and omega-6 fatty acids promote the breakdown of muscle protein and inhibit muscle hypertrophy.

In a document in which data on sarcopenia disease and the intake of omega-3 fatty acid and omega-6 fatty acid were searched and statistically analyzed (Non Patent Literature 2), it is reported that a negative correlation was found between omega-3 fatty acid intake and the incidence of sarcopenia, and a relationship between omega-3 fatty acid intake and sarcopenia has been reported. In addition, Non Patent Literature 3 considers sarcopenia to be one of inflammatory diseases, and teaches that the anti-inflammatory action of omega-3 fatty acid could be a potential therapeutic drug for sarcopenia. However, the effect thereof remains unclear.

As shown above, there are reports stating that 3-hydroxyisovaleric acid, amino acid metabolites, and peptides have the effect of inhibiting muscle atrophy, and reports suggesting the effects of omega-3 fatty acids on sarcopenia. However, there have been no reports to date stating that long-chain hydroxylated fatty acids inhibit muscle atrophy.

### [Citation List]

### [Patent Literature]

[Patent Literature 1]
   JP 2016-88844 A
[Patent Literature 2]
   JP 2019-530743 A
[Patent Literature 3]
   WO2018/105550
[Patent Literature 4]
   JP 2019-172614 A
[Patent Literature 5]
   JP 2022-162858 A

### [Non Patent Literature]

[Non Patent Literature 1]
   Darren G. et al., Biogerontology (2012) 13:345-358
[Non Patent Literature 2]
   Yi Zhang et al., Frontiers in Nutrition, January 2022, Volume 8, Article 738083
[Non Patent Literature 3]
   Ahmed Al Saedi et al., Bone 164 (2022) 116539

### [Summary of Invention]

### [Technical Problem]

The present invention aims to provide a functional fatty acid for inhibiting muscle atrophy, a medicament for improving health status by inhibiting muscle atrophy due to cast immobilization, aging, intake of high-fat diets, metabolic abnormality, nerve disease, cancer, and the like, an improving method, and the like as embodiments thereof.

### [Solution to Problem]

In view of the above-mentioned problem, the present inventors have conducted intensive studies and found that the administration of long-chain hydroxylated fatty acid inhibits muscle atrophy in mice with immobilized hind limbs, that were used as a representative muscle atrophy model. Furthermore, they have also found that the administration of long-chain hydroxylated fatty acid inhibits muscle atrophy induced in mice fed a high-fat diet. In addition, they have found that administration of a hydroxylated fatty acid with 18 carbon atoms and a hydroxyl group at the 10th position, from among the long-chain hydroxylated fatty acids, was highly effective in inhibiting muscle atrophy, which resulted in the completion of the present invention.

The present invention provides the following as specific embodiments. However, the present invention is not limited to these embodiments.
[1] A muscle atrophy inhibitor comprising a long-chain hydroxylated fatty acid as an active ingredient.
[2] The muscle atrophy inhibitor of [1], wherein the long-chain hydroxylated fatty acid has 18 carbon atoms.
[3] The muscle atrophy inhibitor of [2], wherein the long-chain hydroxylated fatty acid has a hydroxyl group at the 10th position.
[4] The muscle atrophy inhibitor of [3], wherein the long-chain hydroxylated fatty acid having a hydroxyl group at the 10th position is 10-hydroxy-cis-12-octadecenoic acid.
[5] The muscle atrophy inhibitor of any of [1] to [4], which is used to prevent or treat a disease accompanied by muscle atrophy.
[6] The muscle atrophy inhibitor of any of [1] to [5], which is used to suppress muscle mass loss.
[7] The muscle atrophy inhibitor of [6], wherein the muscle mass loss is caused by a decrease in physical activity due to immobilization or the intake of a high-fat diet.
[8] The muscle atrophy inhibitor of [5], wherein the disease is sarcopenia, disuse muscle atrophy, muscle atrophy due to obesity, muscle atrophy due to diabetes, myopathy, cachexia, a metabolic disorder, a neurological (nerve) disease, or cancer.
[9] The muscle atrophy inhibitor of [8], wherein the myopathy is muscular dystrophy, distal myopathy, congenital myopathy, a glycogen storage disease, mitochondrial myopathy, steroid myopathy, alcoholic myopathy, rhabdomyolysis, or myasthenia gravis.
[10] The muscle atrophy inhibitor of any of [1] to [9], which is a pharmaceutical product (particularly for human).
[11] A composition for suppressing muscle mass loss, comprising a long-chain hydroxylated fatty acid.
[12] The composition of [11], wherein the long-chain hydroxylated fatty acid has 18 carbon atoms.
[13] The composition of [12], wherein the long-chain hydroxylated fatty acid has a hydroxyl group at the 10-position.
[14] The composition of [13], wherein the long-chain hydroxylated fatty acid having a hydroxyl group at the 10-position is 10-hydroxy-cis-12-octadecenoic acid.
[15] The composition of any of [11] to [14], wherein the muscle mass loss is caused by a decrease in physical activity due to immobilization or intake of a high-fat diet.
[16] The composition of any of [11] to [14], wherein the muscle mass loss is caused by metabolic abnormality, a neurological (nerve) disease, or cancer.
[17] The composition of any of [11] to [14], wherein the composition is a pharmaceutical product.
[18] The composition of any of [11] to [14], wherein the composition is a food or food additive.
[19] The composition of any of [11] to [14], wherein the composition is a feed or feed additive.
[20] A method for inhibiting muscle atrophy in a human or animal, comprising administering an effective amount of a long-chain hydroxylated fatty acid to a subject in need thereof.
[21] A long-chain hydroxylated fatty acid used to inhibit muscle atrophy.
[22] Use of a long-chain hydroxylated fatty acid for the manufacture of a muscle atrophy inhibitor.

### [Advantageous Effects of Invention]

The present invention provides, as an embodiment thereof, a muscle atrophy inhibitor, a composition for suppressing muscle mass loss, and the like, containing a long-chain hydroxylated fatty acid as an active ingredient. These compositions and the like inhibit muscle atrophy resulting from muscle wasting due to a decrease in physical activity by immobilization caused by aging, cast immobilization, and the like, eating habits such as intake of high-fat diets, metabolic abnormalities, neurological (nerve) diseases, cancer, and the like, and can be used in a variety of fields, including pharmaceutical products, foods, feeds, and the like.

### [Brief Description of Drawings]

[Fig. 1]

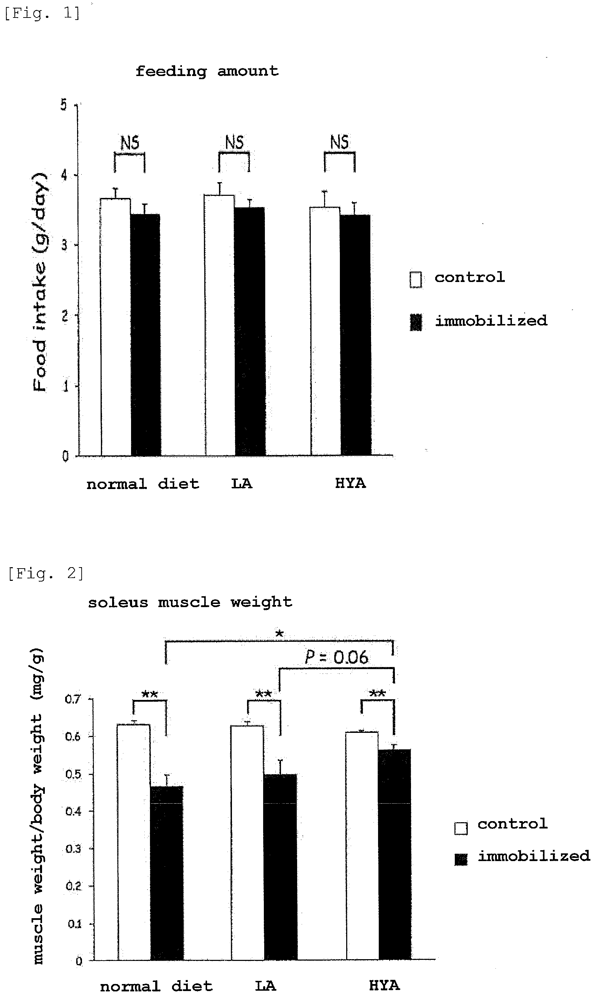

   Fig. 1 shows the amount of food intake when mice were fed a normal diet, a linoleic acid-mixed diet (LA), and a 10-hydroxy-cis-12-octadecenoic acid-mixed diet (HYA) (see Example 1 below).
[Fig. 2]
   Fig. 2 shows the soleus muscle weight after 20 days (including immobilization treatment for the latter 10 days) when mice were fed a normal diet, a linoleic acid-mixed diet (LA), and a 10-hydroxy-cis-12-octadecenoic acid-mixed diet (HYA) (see Example 1 below).
[Fig. 3]

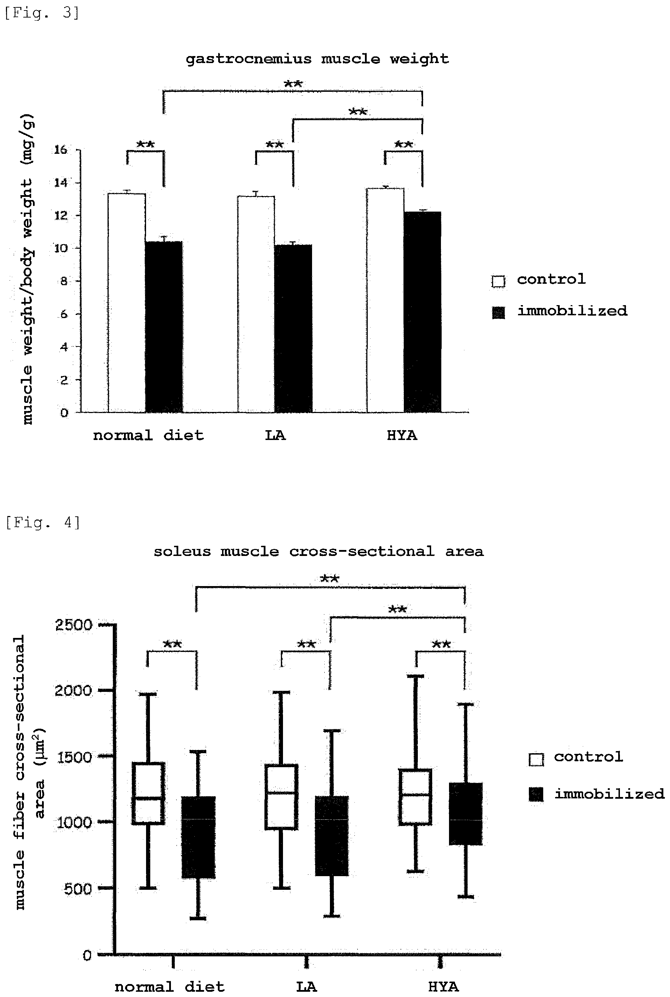

   Fig. 3 shows the gastrocnemius muscle weight after 20 days (including immobilization treatment for the latter 10 days) when mice were fed a normal diet, a linoleic acid-mixed diet (LA), and a 10-hydroxy-cis-12-octadecenoic acid-mixed diet (HYA) (see Example 1 below).
[Fig. 4]
   Fig. 4 shows the cross-sectional area of muscle fibers when the soleus muscle is cut at its largest cross section after 20 days (including immobilization treatment for the latter 10 days) when mice were fed a normal diet, a linoleic acid-mixed diet (LA), and a 10-hydroxy-cis-12-octadecenoic acid-mixed diet (HYA) (see Example 1 below).
[Fig. 5]

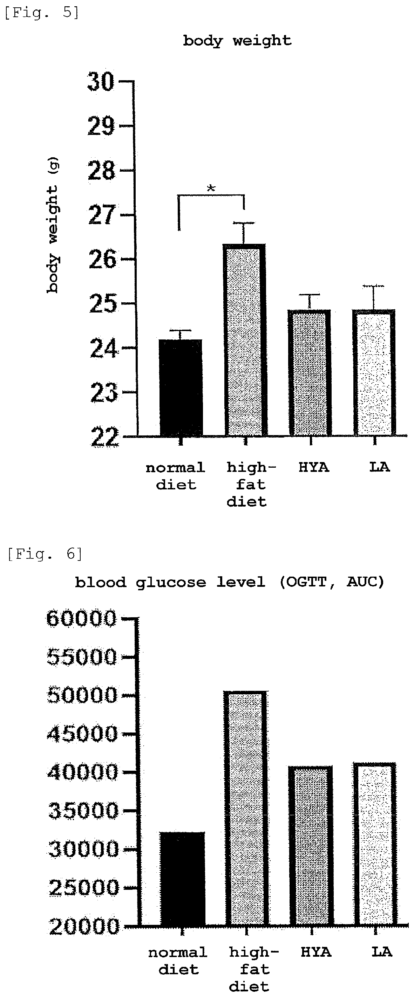

   Fig. 5 shows the results of body weight measurement after 14 days when mice were fed a normal diet, a high-fat diet, a 10-hydroxy-cis-12-octadecenoic acid-added high-fat diet (HYA), or a linoleic acid-added high-fat diet (LA) (see Example 2 below).
[Fig. 6]
   Fig. 6 shows the measurement results of AUC (area under the curve) of blood glucose level after 14 days when mice were fed a normal diet, a high-fat diet, a 10-hydroxy-cis-12-octadecenoic acid-added high-fat diet (HYA), or a linoleic acid-added high-fat diet (LA) (see Example 2 below).
[Fig. 7]

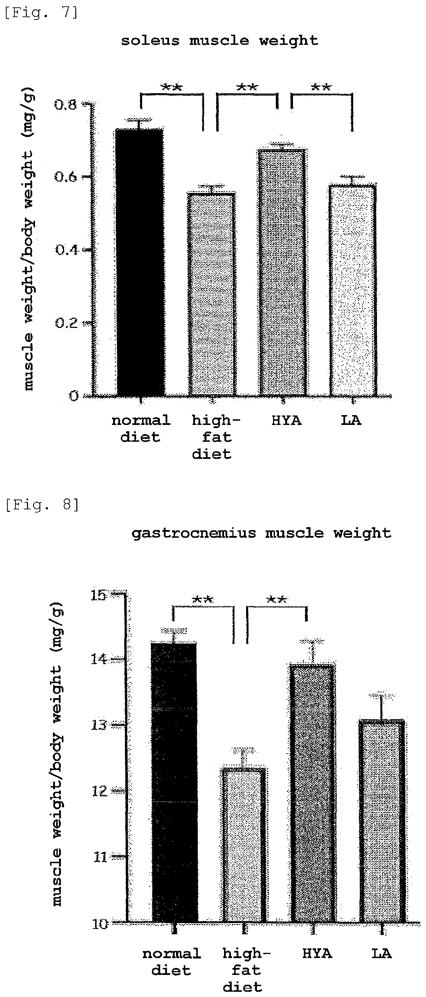

   Fig. 7 shows the soleus muscle weight after 14 days when mice were fed a normal diet, a high-fat diet, a 10-hydroxy-cis-12-octadecenoic acid-added high-fat diet (HYA), or a linoleic acid-added high-fat diet (LA) (see Example 2 below).
[Fig. 8]
   Fig. 8 shows the gastrocnemius muscle weight after 14 days when mice were fed a normal diet, a high-fat diet, a 10-hydroxy-cis-12-octadecenoic acid-added high-fat diet (HYA), or a linoleic acid-added high-fat diet (LA) (see Example 2 below).
[Fig. 9]

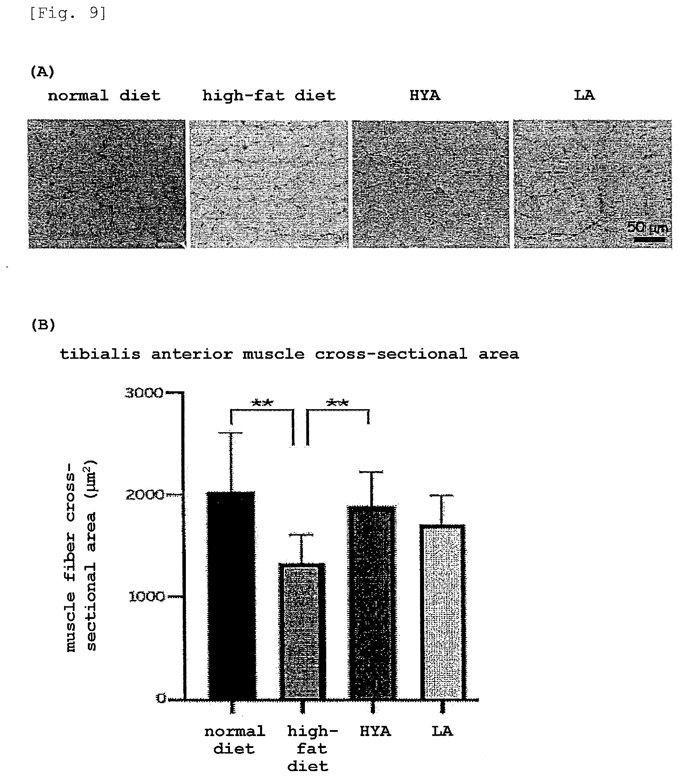

   Fig. 9 shows the results of HE staining of the sections prepared when the tibialis anterior muscle was cut in the cross section at its largest diameter (Fig. (A)), and the comparison results of the cross-sectional area of the tibialis anterior muscle (Fig. (B)), after 14 days when mice were fed a normal diet, a high-fat diet, a 10-hydroxy-cis-12-octadecenoic acid-added high-fat diet (HYA), or a linoleic acid-added high-fat diet (LA) (see Example 2 below).

### [Description of Embodiments]

The present invention is described in detail in the following. Unless otherwise specified in the text, all technical terms and scientific terms used in the present specification have the same meaning as commonly understood by one of ordinary skill in the art to which the present invention belongs. While any methods and materials similar or equivalent to those described in the present specification can be used in the practice or testing of the present invention, the preferred methods and materials are described below.

The contents disclosed in any publication cited herein, including patents and patent applications, are hereby incorporated in their entireties by reference, to the extent that they have been disclosed herein.

The present invention provides muscle atrophy inhibitors, compositions for suppressing muscle mass loss, and the like, which contain long-chain hydroxylated fatty acids. Examples of long-chain hydroxylated fatty acids include fatty acids having 16 to 22 carbon atoms, and fatty acids having 18 carbon atoms are preferred. The hydroxylated fatty acids are preferably hydroxylated fatty acids having a hydroxyl group at the 10-position.

More specifically, the long-chain hydroxylated fatty acids of the present invention include, but are not limited to, 10-hydroxy-cis-12-octadecenoic acid (hereinafter also to be referred to as HYA), 10-hydroxy-cis-12,cis-15-octadecadienoic acid (hereinafter also to be referred to as αHYA), 10-hydroxy-cis-6,cis-12-octadecadienoic acid (hereinafter also to be referred to as γHYA), 10,12-dihydroxyoctadecanoic acid (hereinafter also to be referred to as rHYA), 10-hydroxyoctadecanoic acid (hereinafter also to be referred to as HYB), 10-hydroxy-cis-15-octadecenoic acid (hereinafter also to be referred to as αHYB), 10-hydroxy-cis-6-octadecenoic acid (hereinafter also to be referred to as γHYB), 10-hydroxy-trans-11-octadecenoic acid (hereinafter also to be referred to as HYC), 10-hydroxy-trans-11,cis-15-octadecadienoic acid (hereinafter also to be referred to as αHYC), 10-hydroxy-cis-6,trans-11-octadecadienoic acid (hereinafter also to be referred to as γHYC), 10-hydroxy-cis-6,trans-11,cis-15-octadecatrienoic acid (hereinafter also to be referred to as sHYC), 10,13-dihydroxy-octadecanoic acid (hereinafter also to be referred to as HYE), 10,13-dihydroxy-cis-15-octadecenoic acid (hereinafter also to be referred to as αHYE), 10,13-dihydroxy-cis-6-octadecenoic acid (hereinafter also to be referred to as γHYE), 13-hydroxy-cis-9-octadecenoic acid (hereinafter also to be referred to as HYD), 13-hydroxy-cis-9,cis-15-octadecadienoic acid (hereinafter also to be referred to as αHYD), 13-hydroxy-cis-6,cis-9-octadecadienoic acid (hereinafter also to be referred to as γHYD), 13-hydroxy-cis-6,cis-9,cis-15-octadecatrienoic acid (hereinafter also to be referred to as sHYD), 13-hydroxy-cis-5,cis-9-octadecadienoic acid, 13-hydroxy-trans-5,cis-9-octadecadienoic acid, and the like, and 10-hydroxy-cis-12-octadecenoic acid is preferred.

The long-chain hydroxylated fatty acids of the present invention can be prepared by a known means and, for example, production methods are described in WO2013/168310 and WO2015/111699. In addition, 10-hydroxy-cis-12-octadecenoic acids can be prepared by reference to Biochemical and Biophysical Research Communications 416(2011) p.188-193 and the like.

The muscle atrophy inhibitor and composition for suppressing muscle mass loss containing long-chain hydroxylated fatty acids, which are embodiments of the present invention, are used to prevent or treat muscle atrophy, but the causes of muscle atrophy are not limited. Possible causes of muscle atrophy include a decrease in physical activity caused by cast immobilization due to bone fracture and the like, hospitalization, and aging, eating habits such as intake of high-fat diets and the like, obesity, metabolic abnormality, neurological (nerve) disease, cancer, and the like. Examples of diseases of muscle atrophy caused by them include disuse muscle atrophy, sarcopenia, muscle atrophy due to obesity, muscle atrophy due to diabetes, myopathy, cachexia, and the like.

The long-chain hydroxylated fatty acid, in an embodiment thereof, can be used alone or in the form of a composition formulated as described below (hereinafter collectively referred to as the "composition of the present invention"). The composition of the present invention can be used, for example, as a pharmaceutical product, food, feed, etc., or by blending into these. When using the composition of the present invention, it may be used in combination with other pharmaceutical products, and the like as necessary.

When the composition of the present invention is used as a pharmaceutical product, the dosage form of the pharmaceutical product includes powder, granule, pill, soft capsule, hard capsule, tablet, chewable tablet, rapid-disintegrating tablet, syrup, liquid, suspension, and the like. These formulations are prepared according to conventional methods.

Examples of the additives that can be used for formulation include animal and plant oils such as soybean oil, safflower oil, olive oil, germ oil, sunflower oil, beef fat, sardine oil, and the like, polyvalent alcohols such as polyethylene glycol, propylene glycol, glycerol, sorbitol, and the like, surfactants such as sorbitan ester of fatty acids, sucrose fatty acid ester, glycerin fatty acid ester, polyglycerol fatty acid ester, and the like, excipients such as purified water, lactose, starch, crystalline cellulose, D-mannitol, lecithin, gum arabic, sorbitol solution, carbohydrate solution, and the like, sweetener, colorant, pH adjuster, flavoring agents, and the like. A liquid preparation may be dissolved or suspended in water or other suitable media when in use. Also, tablets and granules may be coated by a well-known method.

When oral administration is difficult, administration as enteral nutrition may be adopted, or a nasogastric tube, gastrostomy tube, or enterostomy tube may be used for administration.

The composition of the present invention may be used as a food or food additive. Examples of foods include, but are not limited to, general foods, health foods, functional foods, nutritional supplements, foods with health claims (e.g., foods for specified health uses, foods with functional claims, and foods with nutritional function), and foods for special dietary uses (e.g., foods for infants, foods for pregnant women, and foods for sick people).

The composition of the present invention may also be provided and sold as a food labeled for use relating to inhibiting muscle atrophy, inhibiting muscle mass loss, and maintaining muscle mass.

Such "labeling" includes all acts intended to inform consumers of the aforementioned intended use, and any expression capable of evoking or inferring the aforementioned intended use falls under the category of "labeling" in this embodiment, regardless of the purpose of the labeling, content of the labeling, labeled object or medium, and the like.

The label is not particularly limited and, for example, a label that appeals inhibition of muscle atrophy, inhibition of muscle mass loss, and muscle maintenance is preferred, and a label of an approved or notified food as necessary (health functional foods such as specified health foods, functional food claims, and nutritional functional foods, foods for special dietary uses, and the like) is more preferred. Examples of the label include "having the function of inhibiting muscle atrophy," "having the function of inhibiting muscle mass loss", "having the function of inhibiting muscle weakness", "having the function of inhibiting muscle wasting", "having the function of supporting the maintenance of muscle mass and strength", "having the function of improving walking ability", "having the function of maintaining walking ability", and the like. Foods approved or notified for functional labeling can be distinguished from general foods.

When the composition of the present invention is used as a food or food additive, the food may be in any form that can be orally ingested, such as solution, suspension, powder, solid molded product, and the like, and is not particularly limited. Specific examples include supplements (powder, granule, soft capsule, hard capsules, tablet, chewable tablet, rapid-integrating tablet, syrup, liquid, etc.), beverages (carbonated drink, lactic acid drink, sports drink, fruit juice drink, vegetable drink, soy milk drink, coffee drink, tea drink, powdered drink, concentrated drink, energy drink, alcoholic drink, etc.), sweets (gummy candy, jelly, gum, chocolate, cookie, candy, caramel, Japanese sweets, snacks, etc.), instant foods (instant noodle, retort pouch food, canned food, microwave food, instant soup/miso soup, freeze-dried food, etc.), oils and fatty foods (mayonnaise, dressing, butter, cream, margarine, etc.), wheat flour products (bread, pasta, noodles, cake mix, breadcrumb, etc.), seasonings (sauce, tomato-processed seasoning, flavor seasoning, cooking mix, soup, etc.), and processed livestock products (livestock ham, sausages, etc.).

The above-mentioned foods can contain, where necessary, various nutrients, various vitamins (vitamin A, vitamin B1, vitamin B2, vitamin B6, vitamin C, vitamin D, vitamin E, vitamin K, etc.), various minerals (magnesium, zinc, iron, sodium, potassium, selenium, etc.), dietary fibers, dispersing agents, stabilizers such as emulsifier and the like, sweetener, flavor components (citric acid, malic acid, etc.), flavoring agents, royal jelly, propolis, Agaricus, and the like.

When the composition of the present invention is used as feed or a feed additive, examples of such feed include pet food, livestock or aquaculture feed additives, and the like.

The subjects to which the composition of the present invention is administered or ingested include humans and animals other than human (e.g., dog, cat, mouse, rat, hamster, guinea pig, rabbit, pig, cow, chicken, parakeet, mynah bird, goat, horse, sheep, monkey, etc.).

The amount of administration or intake of the composition of the present invention varies depending on the subject of administration or intake, target disease, symptoms, route of administration, and the like. For example, the long-chain hydroxylated fatty acid contained in the composition of the present invention can be administered or ingested orally or parenterally at a daily dose of 0.1 to 100 mg/kg body weight, preferably 0.2 to 50 mg/kg body weight, more preferably 0.5 to 30 mg/kg body weight. Administration or intake may be divided into multiple portions per day. It may be increased or decreased depending on the symptoms.

The present invention is explained in more detail in the following by referring to Examples. , The Examples are mere exemplifications of the present invention and do not limit the scope of the present invention in any manner.

### [Example 1]

### Effects of hydroxylated fatty acid on muscle atrophy model mice (immobilized mice)

C57BL/6J mice (male, 10 weeks old) were fed a normal diet (CE-2, CLEA Japan, Inc.), a 1% linoleic acid-containing diet (LA), or a 1% 10-hydroxy-cis-12-octadecenoic acid-containing diet (HYA) (LA manufactured by Sigma-Aldrich and HYA manufactured by Noster Inc. were used) each for 10 days. Then, under general anesthesia, both hind limbs of the mice were immobilized with plastic casts to create immobilized mice. After 10 days of immobilization (still receiving the normal diet or each mixed diet), the mice were sacrificed and weighed. Both soleus muscles were then collected and weighed. The amount of food intake of the normal diet and each mixed diet is shown in Fig. 1. The comparison results of muscle weight per body weight are shown in Fig. 2 (n = 6, *P < 0.05, **P < 0.01, 2-way ANOVA with Bonferroni's post hoc test). Furthermore, under the same conditions, both gastrocnemius muscles were collected and weighed. The comparison results of gastrocnemius muscle weight per body weight are shown in Fig. 3 (n = 6, *P < 0.05, **P < 0.01, 2-way ANOVA with Bonferroni's post hoc test).

In addition, the formalin-soaked soleus muscles were cut in the cross section at its largest diameter, and sections were prepared. After HE staining, the cross-sectional area of the muscle fibers was measured. The comparison results of soleus muscle cross-sectional area are shown in Fig. 4 (the area of 400 fibers pooled from 2 mice was measured and averaged for each condition, **P < 0.01, 2-way ANOVA with Bonferroni's post hoc test).

There were no significant differences in the amount of food intake between the mice in each treatment group. In immobilized model mice, administration of HYA significantly inhibited the loss of soleus muscle, gastrocnemius muscle, and soleus muscle cross-sectional area compared to administration of a normal diet. Similarly, in immobilized model mice, administration of HYA significantly inhibited the loss of gastrocnemius muscle and soleus muscle cross-sectional area compared to administration of linoleic acid. On the other hand, administration of linoleic acid in immobilized model mice did not significantly inhibit the loss of soleus muscle, gastrocnemius muscle, and soleus muscle cross-sectional area compared to administration of a normal diet.

### [Example 2]

### Effects of hydroxylated fatty acid on muscle atrophy model mice (high-fat diet administered mice)

C57BL/6J mice (male, 10 weeks old) were fed a normal diet (CE-2, CLEA Japan, Inc.), a high-fat diet (HFD32, CLEA Japan, Inc.), a 1% HYA-added high-fat diet, or a 1% LA (linoleic acid)-added high-fat diet (HYA manufactured by Noster Inc., LA manufactured by Sigma-Aldrich were used) each for 14 days to create a muscle atrophy model mouse by high-fat diet administration, and the effects by the administration of hydroxylated fatty acid were evaluated.

### (1) Influence of high-fat diet intake on body weight, etc.

The body weight measurement results for each administration group after the above-mentioned 14 days of feeding are shown in Fig. 5 (n = 12, *P < 0.05, 2-way ANOVA with Bonferroni's post hoc test). By this test, it was shown that the body weight increased significantly in the high-fat diet intake group compared to the normal diet intake group.

In addition, an OGTT (oral glucose tolerance test) was conducted on each administration group after the above-mentioned 14 days of feeding. The AUC (area under the curve) of blood glucose level is shown in Fig. 6 (n = 5). By this test, it was shown that the blood glucose level tended to increase greatly in the high-fat diet intake group compared to the normal diet intake group, although the difference was not significant.

The above test results suggest that the muscle atrophy model mouse by the administration of a high-fat diet is suitable for evaluating, for example, "muscle atrophy due to obesity", "muscle atrophy due to diabetes", "muscle atrophy due to metabolic abnormality", and the like.

### (2) Effects of hydroxylated fatty acid

C57BL/6J mice (male, 10 weeks old) were fed a normal diet (CE-2, CLEA Japan, Inc.), a high-fat diet (HFD32, CLEA Japan, Inc.), a 1% HYA-added high-fat diet, or a 1% LA (linoleic acid)-added high-fat diet each for 14 days. After feeding, the mice were sacrificed and weighed. Both soleus muscles were collected and weighed. The comparison results of soleus muscle weight per body weight are shown in Fig. 7 (n = 12, **P < 0.01, 2-way ANOVA with Bonferroni's post hoc test). Furthermore, under the same conditions, both gastrocnemius muscles were collected and weighed. The comparison results of gastrocnemius muscle weight per body weight are shown in Fig. 8 (n = 12, **P < 0.01, 2-way ANOVA with Bonferroni's post hoc test).

In addition, the formalin-soaked tibialis anterior muscle was cut in the cross section at its largest diameter, and sections were prepared. After HE staining (Fig. 9(A)), the cross-sectional area of the muscle fibers was measured. The comparison results of tibialis anterior muscle cross-sectional area are shown in Fig. 9(B) (the area of 400 fibers pooled from 2 mice was measured and averaged for each condition, **P < 0.01, 2-way ANOVA with Bonferroni's post hoc test).

In the high-fat diet-administered mice, the soleus muscle weight, gastrocnemius muscle weight, and tibialis anterior muscle cross-sectional area all significantly decreased due to high-fat diet administration, compared to the case of normal diet administration.

In contrast, the concomitant administration of HYA significantly increased all of the soleus muscle weight, gastrocnemius muscle weight, and tibialis anterior muscle cross-sectional area compared to the case of administration of a high-fat diet alone, demonstrating a remarkable improving effect.

On the other hand, when linoleic acid was administered in combination, a tendency toward an increase was found in all of the soleus muscle weight, gastrocnemius muscle weight, and tibialis anterior muscle cross-sectional area compared to the case of administration of a high-fat diet alone, but the increase was not significant.

While the present invention has been described with emphasis on preferred embodiments, it is obvious to those skilled in the art that the preferred embodiments may be modified.

### [Industrial Applicability]

The present invention has clarified that long-chain hydroxylated fatty acids have an effect of inhibiting muscle atrophy as a conventionally unknown physiological function. A composition for inhibiting muscle atrophy containing such long-chain hydroxylated fatty acid is applicable to a variety of fields, including pharmaceutical products, foods, feeds, and the like, and the present invention is extremely useful industrially.

This application is based on a patent application No. 2023-140522 filed in Japan (filing date: August 30, 2023), the contents of which are incorporated in full herein.

## Claims

1. A muscle atrophy inhibitor comprising a long-chain hydroxylated fatty acid as an active ingredient.

2. The muscle atrophy inhibitor according to claim 1, wherein the long-chain hydroxylated fatty acid has 18 carbon atoms.

3. The muscle atrophy inhibitor according to claim 2, wherein the long-chain hydroxylated fatty acid has a hydroxyl group at the 10th position.

4. The muscle atrophy inhibitor according to claim 3, wherein the long-chain hydroxylated fatty acid having a hydroxyl group at the 10th position is 10-hydroxy-cis-12-octadecenoic acid.

5. The muscle atrophy inhibitor according to any one of claims 1 to 4, which is used to prevent or treat a disease accompanied by muscle atrophy.

6. The muscle atrophy inhibitor according to any one of claims 1 to 4, which is used to suppress muscle mass loss.

7. The muscle atrophy inhibitor according to claim 6, wherein the muscle mass loss is caused by a decrease in physical activity due to immobilization or the intake of a high-fat diet.

8. The muscle atrophy inhibitor according to claim 5, wherein the disease is sarcopenia, disuse muscle atrophy, muscle atrophy due to obesity, muscle atrophy due to diabetes, myopathy, cachexia, a metabolic disorder, a neurological (nerve) disease, or cancer.

9. The muscle atrophy inhibitor according to claim 8, wherein the myopathy is muscular dystrophy, distal myopathy, congenital myopathy, a glycogen storage disease, mitochondrial myopathy, steroid myopathy, alcoholic myopathy, rhabdomyolysis, or myasthenia gravis.

10. The muscle atrophy inhibitor according to any one of claims 1 to 4, which is a pharmaceutical product.

11. A composition for suppressing muscle mass loss, comprising a long-chain hydroxylated fatty acid.

12. The composition according to claim 11, wherein the long-chain hydroxylated fatty acid has 18 carbon atoms.

13. The composition according to claim 12, wherein the long-chain hydroxylated fatty acid has a hydroxyl group at the 10-position.

14. The composition according to claim 13, wherein the long-chain hydroxylated fatty acid having a hydroxyl group at the 10-position is 10-hydroxy-cis-12-octadecenoic acid.

15. The composition according to any one of claims 11 to 14, wherein the muscle mass loss is caused by a decrease in physical activity due to immobilization or intake of a high-fat diet.

16. The composition according to any one of claims 11 to 14, wherein the muscle mass loss is caused by metabolic abnormality, a neurological (nerve) disease, or cancer.

17. The composition according to any one of claims 11 to 14, wherein the composition is a pharmaceutical product.

18. The composition according to any one of claims 11 to 14, wherein the composition is a food or food additive.

19. The composition according to any one of claims 11 to 14, wherein the composition is a feed or feed additive.
